# EUROPEAN PATENT APPLICATION

(11) **EP 2 269 575 A1**
(43) Date of publication of application: **05.01.2011**
(21) Application number: 09397519.1
(22) Date of filing: 30.06.2009
(51) Int. Cl.: A61K 9/00, A61K 47/18, A61K 31/5575

(54) **Method for improving bioavailability of latanoprost**

(71) Applicant: Santen Pharmaceutical Co., Ltd, Osaka 533-8651 (JP)
(72) Inventor: Reunamäki, Timo, FI-33820, Tampere (FI); Tervo, Paula, FI-33900, Tampere (FI); Lokkila, Jukka, FI-36110, Ruutana (FI); Pellinen, Pertti, FI-33880, Lempäälä (FI); Alajuuma, Päivi, FI-37600, Valkeakoski (FI); Oksala, Olli, FI-33240, Tampere (FI)
(74) Representative: Matilainen, Mirja Helena

(57) **Abstract**

The present invention relates to a method for improving ocular bioavailability of latanoprost by adding an organic amine to an aqueous eye drop solution containing latanoprost. The invention further relates to an aqueous eye drop composition in which a better ocular bioavailability of latanoprost is achieved by adding an organic amine to the aqueous eye drop solution containing latanoprost, and to a method for treating ocular hypertension and glaucoma by administering said composition to a subject in need of such treatment.

## Description

### Field of the invention

The present invention relates to a method for improving ocular bioavailability of latanoprost by adding an organic amine to an aqueous eye drop solution containing latanoprost. The invention further relates to an aqueous eye drop composition in which a better ocular bioavailability/penetration of latanoprost is achieved by adding an organic amine to the aqueous eye drop solution containing latanoprost, and to a method for treating ocular hypertension and glaucoma by administering said composition to a subject in need of such treatment.

### Background

Like other prostaglandin derivatives, such as isopropyl unoprostone, tafluprost or travoprost, latanoprost is a highly lipophilic medicament. The concentration of latanoprost used for the treatment of glaucoma is very low, approximately 0.005 % (w/v). However, although latanoprost is effective in low amounts, there is still a need to reduce that minor amount to its effective minimum to be administered onto the eye surface. On the other hand, when the amount of the active agent in an eye drop composition is lowered, a balanced but sufficient penetration may become critical or impossible to obtain repeatedly and safely.

Adjuvant agents are included in aqueous eye drop solutions in order to achieve sufficiently stable and effective eye drops for the desired purposes. Preservatives have been included in ophthalmic compositions not only for their antimicrobial effect but also for their stabilising and homogenizing interactions with the other ingredients. However, preservatives are also known as the major etiology of keratoconjuctive disorders, and for safety purposes it is preferred that the concentration of preservatives, such as those of benzalkonium chloride (BAK) type, is as low as possible. Animal studies and in vitro studies have demonstrated that preservatives such as BAK cause harmful effects on several eye tissues, including the tear film, cornea, conjunctiva and trabecular cells (Baudouin, 2008). Evidence from some rabbit and human studies has also suggested that the presence of BAK may affect the corneal epithelium layer and thus increase the transcorneal penetration of poorly permeable drugs. However, a recent study (Pellinen and Lokkila, 2009) has shown that the corneal penetration into rabbit aqueous humor is comparable between BAK-preserved and preservative-free tafluprost, which is another member in the family of prostaglandin derivatives. The dual role of preservatives (both antimicrobial and stabilizing/solubilizing effects) has lead to difficulties e.g. in preparing stable, preservative-free unit dose eye drop preparations.

Nonionic surfactants and antioxidants have been added to ophthalmic aqueous prostaglandin derivative solutions e.g. in order to prevent lowering of concentration of said prostaglandin derivatives. The latanoprost composition of WO2004/037267 (Sucampo) comprises polysorbate or polysorbate and EDTA, but substantially no BAK. In EP 1321144 (Santen), nonionic surfactants, especially polysorbates, have been found to prevent a prostaglandin derivative from being adsorbed to resinous container walls. The use of nonionic surfactants has been almost inavoidable in ophthalmic aqueous compositions comprising prostaglandin derivatives, especially if a preservative-free aqueous composition has been desired to be produced.

Organic amines have been used in several ophthalmic compositions, mostly as buffers and pH adjusting agents. JP03/146881 (Toyo) discloses the prevention of precipitation of anti-allergic pemirolast potassium using trometamol as buffering agent. According to JP03/327530 (Sankyo) the preservative effect of a cationic can be enhanced with trometamol. Incorporating trometamol with a non-ionic surfactant into a suspension-type preparation of an oxazine-picolinic acid has been found to result in suppression of the adhesion of the active ingredient (WO08/111630, Santen). An antiseptic ophthalmic composition free from irritation to eyes can be formulated by including i.a. trometamol in the composition (JP04/002364, Lion; JP03/300871, Lion and JP03/206241, Teika). According to US03/055052 (Wakamoto) an excellent but less stimulative ophthalmic antiinflammatory effect can be obtained by adding trometamol to an ophthalmic preparation. In JP99/11302-162 (Kissei), a better anti-allergic effect of the active ingredient is obtained by incorporating trometamol. Promotion of intraocular penetration of a phenylacetic acid NSAID-compound is possible from a composition containing trometamol (EP 1 808 170, Senju). According to EP 1 916 002 (Santen), degradation of a thermally unstable medicament, such as latanoprost, can be prevented by adding an organic amine such as trometamol.

However, despite of the above mentioned several references of eye drop formulations and various organic amines, there is no disclosure or suggestion that adding an organic amine to an ophthalmic aqueous latanoprost composition could cause a better transcorneal penetration and hence improved ocular bioavailability in relation to the reduced administered amount.

### Summary of the invention

The present invention relates to a method for improving ocular bioavailability of preservative-free latanoprost in an aqueous eye drop composition by adding an organic amine to the eye drop composition containing latanoprost.

Another object of the invention is an aqueous preservative-free eye drop composition comprising latanoprost, wherein the ocular bioavailability of latanoprost is improved by adding an organic amine to the latanoprost containing eye drop.

A further object of the invention is a method for treating ocular hypertension and glaucoma by administering to a subject in need of such treatment an effective amount of an aqueous preservative-free eye drop composition comprising latanoprost, wherein the ocular bioavailability of latanoprost is improved by adding an organic amine to the latanoprost containing eye drop.

Within this description, "substantially no preservatives" or "preservative-free" means that the solution contains absolutely no preservatives, or the solution contains preservatives at a concentration that is undetectable or does not provide a preservative effect.

"Bioavailability" refers to the degree and rate at which a drug is absorbed and made available in the target tissue. "Ocular bioavailability" refers more specifically to the concentration of a drug in the aqueous humor after topical administration of an ophthalmic solution.

"Stability" refers to chemical and physical stability of the ophthalmic solution containing a drug.

### Brief description of the figures

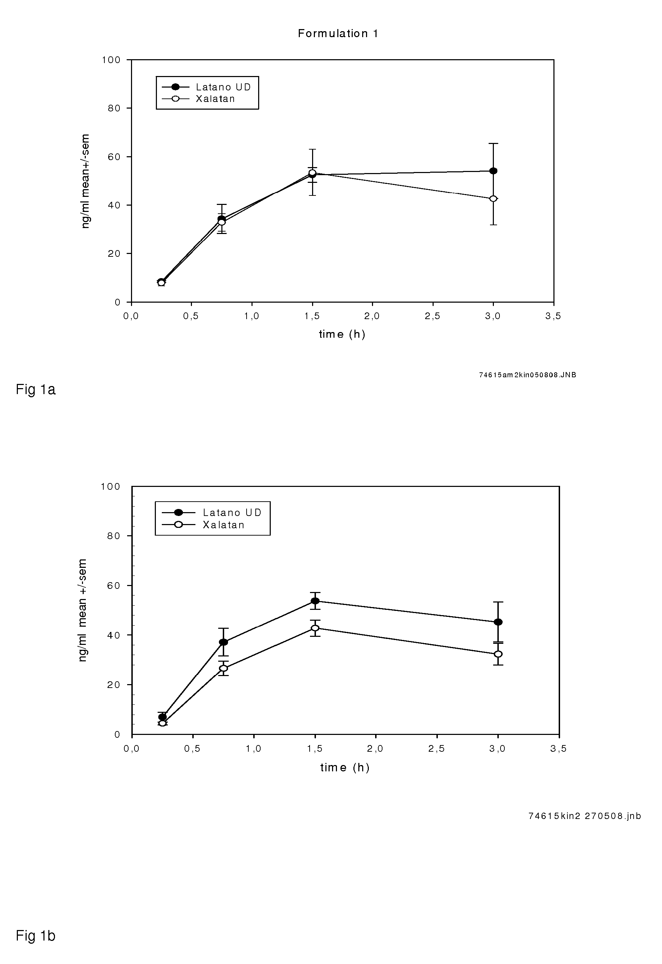

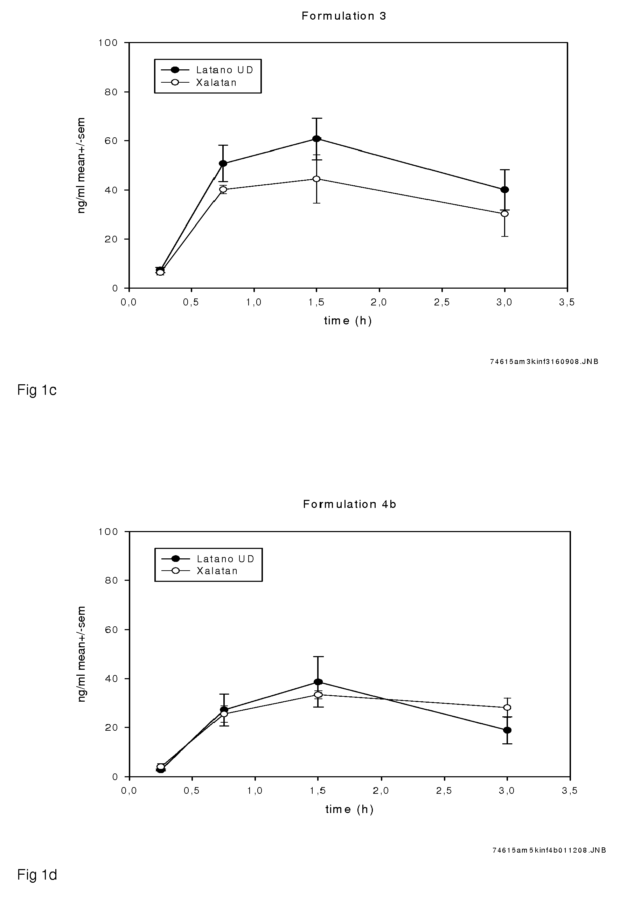

Figures 1a-1c illustrate absorption of latanoprost from preservative-free test solutions with different latanoprost-trometamol ratio into aqueous humor of rabbit eye in comparison with commercial Xalatan, when latanoprost concentration in the test solution was the same as in Xalatan. In figure 1d, latanoprost concentration in the test solution was 70 % of the latanoprost concentration in Xalatan.

The latanoprost-trometamol ratios used in the test solutions were as follows:
Fig 1a: Latanoprost - Trometamol ratio: 0.013
Fig 1b: Latanoprost - Trometamol ratio: 0.017
Fig 1c: Latanoprost - Trometamol ratio: 0.020
Fig 1d: Latanoprost - Trometamol ratio: 0.029

Figures 2a - 2c show intraocular pressure after topical administration in rats when preservative-free test solutions with different latanoprost-trometamol ratios and different latanoprost concentrations were used in comparison with commercial Xalatan.
The latanoprost-trometamol ratios used in the test solutions were as follows:

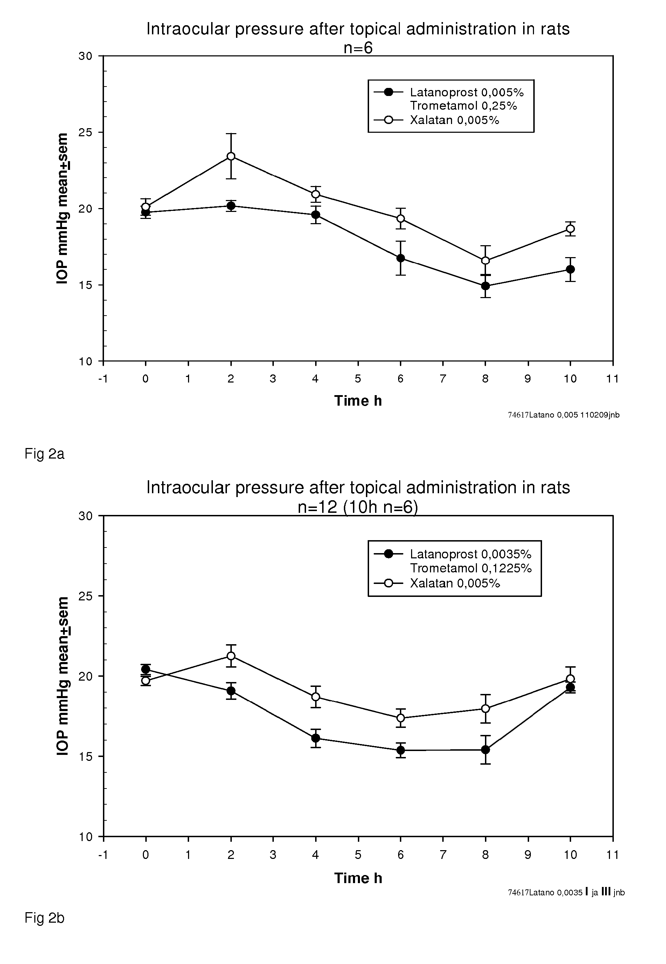

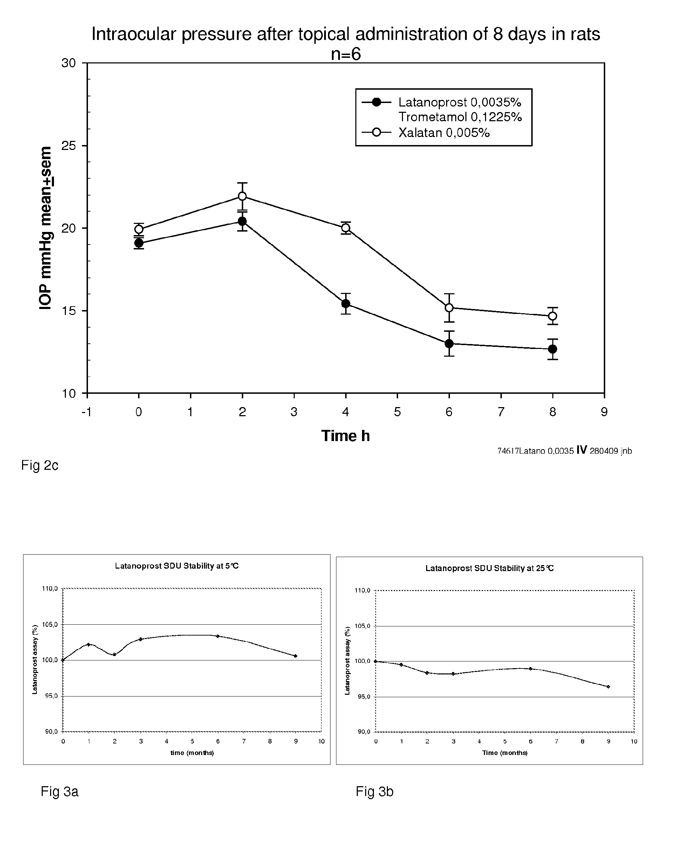

Fig 2a: Latanoprost - Trometamol ratio: 0.020 (latanoprost concentration the same as in Xalatan)
Fig 2b-2c: Latanoprost - Trometamol ratio: 0.029 (latanoprost concentration 70% of Xalatan)
Figures 3a and 3b show preliminary stability of latanoprost unit dose eye drop composition during storage at 5 °C and at 25 °C, respectively.

### Description of the invention

According to the present invention it has now been found that ocular bioavailability of latanoprost in an aqueous eye drop composition can be improved by adding an organic amine to the aqueous eye drop composition containing latanoprost. This invention enables to provide homogenous and stable, preservative-free aqueous ophthalmic latanoprost solution as a sterile, preferably unit dose type formulation. The aqueous ophthalmic latanoprost solution according to the invention is evidently also less irritative to the eye than the currently available commercial product.

The ingredients other than latanoprost and an organic amine may not be particularly limited as far as latanoprost is homogenously and stably dissolved in the aqueous solution. However, it is notable that nonionic surfactants (polysorbates, cremophores etc.) or preservatives are not required in the aqueous ophthalmic latanoprost solution of the invention to provide a stable and bioavailable formulation for use in a single dose form. The aqueous ophthalmic solution of the present invention also remains stable at room temperature, contrary to the currently available commercial product which requires cold storage (at 2-8 °C).

The concentration of latanoprost in the ophthalmic aqueous solution and dosing frequency may vary according to the type and condition of the subject to be treated. For example, an ophthalmic solution containing latanoprost at a concentration of 0.01 to 0.0005 %, preferably 0.0075 to 0.001 %, more preferably 0.005 to 0.002 % (w/v) may be instilled 1 to 4 times, preferably 1 to 2 times, and more preferably 1 time a day. As the invention provides improved ocular bioavailability and IOP lowering effect of latanoprost, the amount of latanoprost in the aqueous ophthalmic solution can be lowered compared to the prior art products, still achieving an effective product.

The organic amine is a water-soluble organic amine, examples of which include organic amines having a hydroxy group, such as monoethanolamine, diethanolamine, triethanolamine, trometamol and meglumine. A preferred organic amine is trometamol. The concentration of the organic amine is for example in the case of trometamol preferably in the range of from 0.01 % to 1 % (w/v), more preferably from 0.05 to 0.75 % (w/v).

The ophthalmic solution according to the invention may also comprise conventional excipients used in ophthalmic compositions, such as buffering agents, solvents, pH adjusters, tonicity agents, viscocity enhancers and the like. Preferred excipients for use in the ophthalmic solution of the present invention are viscosity enhancers. Suitable viscosity enhancers include but are not limited to cellulose derivatives, such as hydroxypropyl methylcellulose, sodium carboxymethylcellulose, methylcellulose, polyvinylpyrrolidone, polyvinylalcohols, dextrans and polyacrylic acids. Examples of suitable buffering agents include but are not limited to sodium dihydrogen phosphate dihydrate, boric acid, borax, citric acid, or ε-aminocaproic acid. Specific examples of tonicity agents include but are not limited to glycerol, sorbitol, mannitol and other sugar alcohols, propylene glycol, sodium chloride, potassium chloride and calcium chloride

The pH of the ophthalmic aqueous solution according to the invention is preferably from 4 to 8, more preferably from 5 to 7. As pH adjusters, common pH adjusting agents such as sodium hydroxide and/or hydrochloric acid may be used.

The ophthalmic solution according to the invention is preferably packaged in a container consisting essentially of polyethylene or in contact with material consisting essentially of polyethylene. The container material may contain minor amounts of other materials than polyethylene, for example polypropylene, polyethylene terephthalate, polyvinyl chloride, acrylic resins, polystyrene, polymethyl methacrylate and nylon 6. The amount of said materials is preferably no more than about 5 to 10 % of the total container material.

Containers for packaging and storing the aqueous ophthalmic solution according to the invention include all container forms suitable for user-friendly topical ophthalmic delivery, preferably those of unit dose form. Consequently, the containers may be selected for example from the group consisting of bottles, tubes, ampoules, pipettes and fluid dispensers for dispensing minute amounts of sterile fluid.

The containers for the ophthalmic solution according to the invention are preferably manufactured by extrusion blow moulding method. Extrusion blow moulding gives smoother inner surface of the container compared to injection blow moulding, which is commonly used to manufacture for example polyethylene multidose bottles. The smoother inner surface gives better chemical stability of prostaglandins in the polyethylene container compared to polyethylene container manufactured by injection blow moulding. Furthermore, when single-dose containers are used, they are sterilized during the moulding process by heat and no additional sterilization of containers is needed, which also improves stability of prostaglandins in a single-dose container. Conventional techniques for sterilization of prostaglandin eye drop products consist of treatment of the empty plastic bottles with highly toxic ethylene oxide gas before filling-in the solution (see EP 1 825 855, Santen) and EP 1 349 580, Novartis). The much smoother sterilization process used in the preparation of the eye drop composition of the present invention (heat sterilization during the filling process) is however more recommendable for a unit dose preparation especially in polyethylene containers.

Although unit dose containers are preferred for the purposes of the invention, the aqueous ophthalmic solution according to the invention remains soluble, stable and bioavailable also in fluid dispensers for dispensing of minute amounts of germ-free fluid or in any other container-type wherein the aqueous ophthalmic solution is in contact with container material consisting essentially of polyethylene. Such fluid dispensers are disclosed for example in US 5,614,172 (Ursapharm).

The preservative-free aqueous ophthalmic solution according to the invention can be stored at room temperature in the above mentioned suitable containers, including unit dose pipettes and dispensers. Stability studies have shown that a preservative-free aqueous ophthalmic latanoprost solution according to the invention is stable in a polyethylene container for a long time, at least for 9 months at 5 °C and at 25 °C.

The following examples illustrate the invention without limiting the same in any way.

### Example 1.

Male albino rabbits of New Zealand White strain received the ophthalmic solutions described above in connection with Figure 1. In each rabbit, one eye was dosed topically with one of the test solutions and the other eye with the reference solution (commercial Xalatan). The rabbits were sacrificed at each time point (4 animals per treatment per time point) and aqueous humor sample was taken. The concentration of latanoprost acid was measured using the HPLC/MS method.

The results are shown in Figures 1a-1d. It can be seen that by increasing the latanoprost:trometamol ratio in preservative-free test solutions penetration of latanoprost into aqueous humor of rabbit eye is improved.

### Example 2.

Spraque-Dawley rats were used to study the effects of ophthalmic solutions on the intraocular pressure (IOP). The test solutions were dosed topically (5 µl) either as a single instillation or repeated instillations onto one eye and the reference solution (commercial Xalatan) onto the other. Six to twelve animals were used per study. IOP was measured prior to dosing at 0 h and 2, 4, 6 and 8 hours (or additional 10 hours) after the dosing. Measurement was performed with a TonoLab rebound tonometer.

The results are shown in Figures 2a - 2c. From the results it can be seen that the commercial product causes an immediate increase in the IOP and the IOP lowering effect is seen only after several (6 to 8) hours. The compositions according to the invention do not provide an increase in the IOP and the IOP lowering effect starts earlier than with the current commercial product. The IOP lowering effect stays at the same level after repeated instillation as it is after single instillation. Furthermore, the IOP lowering effect of the composition according to the invention is better than that of the commercial product, even with a latanoprost concentration of only 70 % of the amount of latanoprost in the commercial product.

### Example 3.

The stability of preservative-free latanoprost in low density polyethylene containers was studied for 9 months at 5 °C and 25 °C. The composition of the preservative-free aqueous latanoprost formulation in the study was 0.003 % latanoprost, 0.3 % trometamol, 0.9 % sodium chloride and hydrochloric acid / sodium hydroxide to adjust the pH to 7.0.

0.3 ml of the composition prepared above was filled in the body part of the polyethylene unit dose container and sealed by heating with the upper part of the container. The containers were packaged into paper coated aluminium-polyethylene foil and stored in refrigerator or incubator.

The concentration of latanoprost was measured by HPLC/UV. The results in comparison to time zero value are presented in figures 3a-3b. The results show that latanoprost concentration remains at the same level at 5 °C and 25 °C at least for nine (9) months.

### References

Baudouin C. Detrimental effect of preservatives in eyedrops: implications for the treatment of glaucoma. Acta Ophthalmol 2008; 86:716-726.

Pellinen P, Lokkila. Corneal penetration into rabbit aqueous humor is comparable between preserved and preservative-free tafluprost. Ophthalmic Res 2009; 41:118-122.

## Claims

1. A method for improving ocular bioavailability of latanoprost from an aqueous eye drop composition by adding an organic amine to the eye drop composition containing latanoprost.

2. The method according to claim 1, wherein the organic amine is an organic amine having a hydroxy group.

3. The method according to claim 2, wherein the organic amine having a hydroxy group is trometamol.

4. The method according to claim 1, wherein the aqueous latanoprost eye drop composition contains substantially no preservatives.

5. The method according to claim 1, wherein the aqueous latanoprost eye drop composition contains viscosity enhancing agents.

6. An aqueous eye drop composition comprising latanoprost, wherein the ocular bioavailability of latanoprost is improved by adding an organic amine to the latanoprost containing eye drop.

7. The aqueous eye drop composition according to claim 6 wherein the organic amine is an organic amine having a hydroxy group.

8. The aqueous eye drop composition according to claim 7, wherein the organic amine having a hydroxy group is trometamol.

9. The aqueous eye drop composition according to claim 7 which contains substantially no preservatives or non-ionic surfactants.

10. The aqueous eye drop composition according to claim 6 which further comprises viscosity enhancing agents.

11. The aqueous eye drop composition according to claim 6, which comprises 0.0025 to 0.005 % (w/v) latanoprost, 0.1 to 1 % (w/v) trometamol, optionally buffering agents, pH adjusters, tonicity and viscosity enhancing agents conventionally used in ophthalmic solutions, and substantially no preservatives, in a unit dose container or fluid dispenser consisting essentially of polyethylene or in contact with container material consisting essentially of polyethylene.

12. A method for treating ocular hypertension and glaucoma by administering to a subject in need of such treatment an effective amount of an aqueous eye drop composition comprising latanoprost, wherein the ocular bioavailability of latanoprost is improved by adding an organic amine to the latanoprost containing eye drop.
